# EUROPEAN PATENT APPLICATION

(11) **EP 3 517 042 A1**
(43) Date of publication of application: **31.07.2019**
(21) Application number: 19150539.5
(22) Date of filing: 07.01.2019
(51) Int. Cl.: A61B 8/00, A61B 8/08

(54) **ULTRASONIC IMAGING APPARATUS**

(30) Priority: 09.01.2018 KR 20180002817
(71) Applicant: Samsung Medison Co., Ltd., Hongcheon-gun, Gangwon-do, 25108 (KR)
(72) Inventor: CHOI, Jin Ho, Gyeonggi-do (KR)
(74) Representative: Grootscholten, Johannes A.M.

(57) **Abstract**

A disclosed ultrasonic imaging apparatus includes a probe to irradiate and receive ultrasonic waves, a correction member including an inspection object contact portion provided to be in contact with an inspection object and to be deformable according to the shape of the inspection object and a probe contact portion provided such that the probe is slidably moved and having a curved surface, a sensor to measure a distance between the probe and the probe contact portion or a force applied to the probe contact portion by the probe, and a probe driving device to slidingly move the probe on the curved surface of the probe contact portion and to move the probe so that the probe is brought in close contact with the probe contact portion corresponding to information measured by the sensor.

## Description

### BACKGROUND

### 1. Technical Field

The present disclosure relates to an ultrasonic imaging apparatus, and more particularly, to an ultrasonic imaging apparatus having an improved inspection method.

### 2. Description of the Related Art

An ultrasonic imaging apparatus is an apparatus that irradiates an ultrasonic signal from a body surface of a target toward a target site in the body and obtains an image of a monolayer or blood flow of soft tissue without invasion by using information of a reflected ultrasonic signal (ultrasonic echo signal).

The ultrasonic imaging apparatus is widely used for the diagnosis of the heart, abdomen, urinary system and obstetrics because it is small, inexpensive, real-time displayable, easy to use, and has high safety because there is no radiation exposure, compared to other imaging apparatuses such as an X-ray diagnostic apparatus, an X-ray CT scanner, an MRI (Magnetic Resonance Image) and a nuclear medicine diagnostic apparatus.

The ultrasonic imaging apparatus may include an ultrasonic probe for transmitting and receiving ultrasonic waves. The ultrasonic probe may transmit ultrasonic waves to a target object through a transducer and receive echoes reflected from the target object.

When an inspector performs an inspection by manipulating the ultrasonic probe, the image quality of the ultrasonic imaging apparatus depends on the proficiency level of the inspector. Further, when a portion of an inspection object formed as a curved surface such as a breast is inspected, multiple scans need to be performed at multiple angles, and if the breast is squeezed, a subject may feel pain.

### SUMMARY

It is an aspect of the present disclosure to provide an ultrasonic imaging apparatus capable of inspecting a portion of an inspection object formed as a curved surface such as a breast by one scan.

It is another aspect of the present disclosure to provide an ultrasonic imaging apparatus capable of inspecting while pressing a portion of an inspection object formed as a curved surface such as a breast with minimal deformation.

It is another aspect of the present disclosure to provide an ultrasonic imaging apparatus capable of reducing a degree of intervention of an inspector to obtain an image of a constant quality.

Additional aspects of the present disclosure will be set forth in part in the description which follows and, in part, will be obvious from the description, or may be learned by practice of the disclosure.

In accordance with an aspect of the present disclosure, an ultrasonic imaging apparatus includes a probe to irradiate and receive ultrasonic waves, a correction member including an inspection object contact portion provided to be in contact with an inspection object and to be deformable according to the shape of the inspection object and a probe contact portion provided such that the probe is slidably moved and having a curved surface, a sensor to measure a distance between the probe and the probe contact portion or a force applied to the probe contact portion by the probe, and a probe driving device to slidingly move the probe on the curved surface of the probe contact portion and to move the probe so that the probe is brought in close contact with the probe contact portion corresponding to information measured by the sensor.

The probe driving device may include a movement guide, a first driving member slidably coupled to the movement guide, and a second driving member rotatably coupled to the first driving member.

The probe driving device may further include a first probe driving device to move the first driving member on the movement guide, and a second probe driving device to rotate the second driving member with respect to the first driving member.

The second probe driving device may be configured to rotate the second driving member so that the probe approaches the probe contact portion when a distance between the probe and the probe contact portion measured by the sensor is longer than a preset distance or when a force applied to the probe contact portion by the probe is smaller than a preset force, and rotate the second driving member so that the probe is away from the probe contact portion when the distance between the probe and the probe contact portion measured by the sensor is shorter than the preset distance or when the force applied to the probe contact portion by the probe is larger than the preset force.

The probe driving device may include a movement guide, a first driving member slidably coupled to the movement guide, and a second driving member slidably coupled to the first driving member.

The probe driving device may further include a first probe driving device to move the first driving member on the movement guide, and a second probe driving device to slide the second driving member with respect to the first driving member.

The second probe driving device may be configured to slide the second driving member so that the probe approaches the probe contact portion when a distance between the probe and the probe contact portion measured by the sensor is longer than a preset distance or a force applied to the probe contact portion by the probe is smaller than a preset force, and slide the second driving member so that the probe is away from the probe contact portion when the distance between the probe and the probe contact portion measured by the sensor is shorter than the preset distance or when the force applied to the probe contact portion by the probe is larger than the preset force.

The probe driving device may include a movement guide formed to have a curvature corresponding to a curvature of the probe contact portion, and a driving member slidably coupled to the movement guide.

The sensor may be disposed in the probe.

The ultrasonic imaging apparatus may further include a fixing member to apply a force to the correction member so that the correction member and the inspection object are brought into close contact with each other.

The fixing member may include a belt connected to the correction member, and a roller provided to be connected to the belt and apply a force to the belt so that the correction member is brought into close contact with the inspection object or apply a force to the belt so that the correction member is apart from the inspection object.

The fixing member may include a tension member connected to the correction member and made of a material having elasticity.

The tension member may include a first tension member having a first connecting portion, and a second tension member having a second connecting portion connected to the first connecting portion of the first tension member.

The inside of the correction member may be filled with a liquid passing ultrasonic waves.

The correction member may include a probe guide disposed at the probe contact portion to guide the movement of the probe and made of a material harder than the probe contact portion.

In accordance with another aspect of the present disclosure, an ultrasonic imaging apparatus includes a probe to irradiate and receive ultrasonic waves, a correction member including an inspection object contact portion provided to be in contact with an inspection object and to be deformable according to the shape of the inspection object and a probe contact portion provided such that the probe is slidably moved and having a curved surface, a probe guide disposed at the probe contact portion to guide the movement of the probe and made of a material harder than the probe contact portion, a sensor to measure a distance between the probe and the probe contact portion or a force applied to the probe contact portion by the probe, a probe driving device to move the probe, and a controller to drive the probe driving device so that the probe is brought into close contact with the probe contact portion corresponding to information measured by the sensor.

The controller may be configured to control the probe driving device to move the probe so as to approach the probe contact portion when a distance measured by the sensor is longer than a preset distance or when a force measured by the sensor is smaller than a preset force, and control the probe driving device to move the probe away from the probe contact portion when the distance measured by the sensor is shorter than the preset distance or when the force measured by the sensor is larger than the preset force.

The ultrasonic imaging apparatus may further include a fixing member to apply a force to the correction member so that the correction member and the inspection object are brought into close contact with each other, wherein the fixing member may include a belt connected to the correction member and a roller device to drive the belt to apply tension to the belt.

The controller may be configured to drive the roller device to apply tension to the belt when the correction member is mounted on the inspection object.

In accordance with another aspect of the present disclosure, an ultrasonic imaging apparatus includes a probe to irradiate and receive ultrasonic waves, a correction member including an inspection object contact portion provided to be in contact with an inspection object and to be deformable according to the shape of the inspection object and a probe contact portion provided such that the probe is slidably moved and having a curved surface, a sensor to measure a distance between the probe and the probe contact portion or a force applied to the probe contact portion by the probe, a first driving member to slidingly move the probe on the curved surface of the probe contact portion, and a second driving member rotatably coupled to the first driving member and provided to move the probe in a direction of approaching the probe contact portion or in a direction of moving away from the probe contact portion.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating an ultrasonic imaging apparatus according to an embodiment of the present disclosure;
FIG. 2 is a view illustrating a state in which a correction member shown in FIG. 1 is mounted on an inspection object;
FIG. 3 is a view illustrating a probe device, the correction member and a fixing member shown in FIG. 1;
FIGS. 4 to 6 are views sequentially illustrating the operation of the probe device shown in FIG. 3;
FIG. 7 is a control block diagram of the probe device shown in FIG. 3;
FIG. 8 is a view showing fixing members according to another embodiment of the present disclosure;
FIGS. 9 to 11 are views sequentially illustrating the operation of a probe device according to another embodiment of the present disclosure; and
FIGS. 12 to 14 are views sequentially illustrating the operation of a probe device according to another embodiment of the present disclosure.

### DETAILED DESCRIPTION

The embodiments described herein and the configurations shown in the drawings are only examples of preferred embodiments of the present disclosure, and various modifications may be made at the time of filing of the present disclosure to replace the embodiments and drawings of the present application.

Like reference numbers or designations in the various drawings of the present application represent parts or components that perform substantially the same functions.

The terms used in this specification are for the purpose of describing the embodiments and are not intended to restrict and/or to limit the disclosure. The singular expressions may include plural expressions, unless the context clearly dictates otherwise. In this specification, the terms "comprises" and "has" are intended to indicate that there are features, numbers, steps, operations, elements, parts, or combinations thereof described in the specification, and do not exclude the presence or addition of one or more other features, numbers, steps, operations, elements, parts, or combinations thereof.

It will be understood that, although the terms first, second, etc. may be used herein to describe various components, these components should not be limited by these terms. These terms are only used to distinguish one component from another. For example, without departing from the scope of the present disclosure, the first component may be referred to as a second component, and similarly, the second component may also be referred to as a first component.

In this specification, the terms "front," "rear," "upper," "lower," "left," and "right" are defined with reference to the drawings, and the shape and position of each component are not limited by these terms.

Hereinafter, embodiments according to the present disclosure will be described in detail with reference to the accompanying drawings.

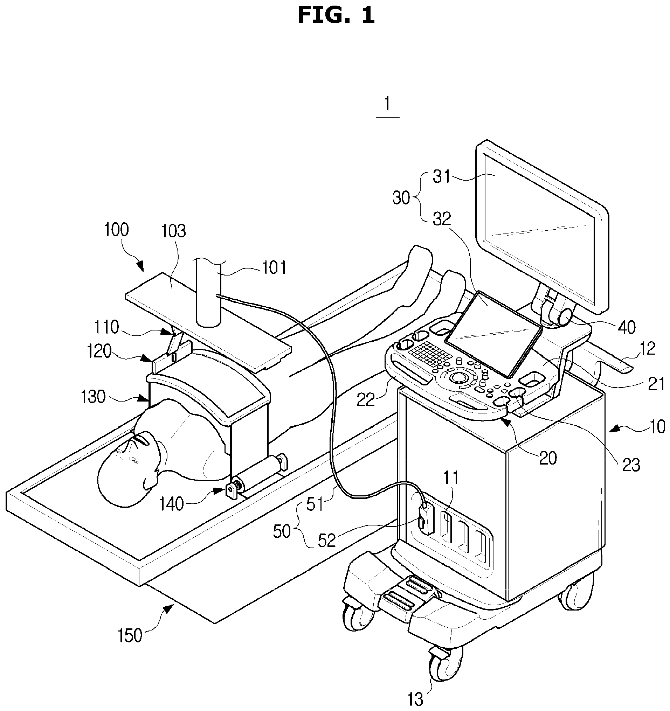

FIG. 1 is a view illustrating an ultrasonic imaging apparatus according to an embodiment of the present disclosure.

Referring to FIG. 1, an ultrasonic imaging apparatus 1 according to an embodiment of the present disclosure may include a main body 10, a controller 20, a display device 30, a probe device 100, and a correction member 130. The display device 30 may include a first display 31 and a second display 32.

The display device 30 may display an ultrasonic image obtained in the ultrasonic diagnostic process. The display device 30 may also display an application related to the operation of the ultrasonic imaging apparatus 1. For example, the first display 31 may display ultrasonic images obtained in the ultrasonic diagnostic process, and the second display 32 may display items related to the operation of the ultrasonic imaging apparatus 1.

The first display 31 or the second display 32 may be implemented as a cathode ray tube (CRT) or a liquid crystal display (LCD). The first display 31 or the second display 32 may be provided to be coupled to the main body 10 and be separated from the main body 10.

The ultrasonic imaging apparatus 1 may further include a display connecting member 40. The display connecting member 40 may connect the main body 10 and the first display 31. The display connecting member 40 may be configured such that the first display 31 may be moved from the main body 10. The first display 31 may be disposed apart from the main body 10 by the display connecting member 40.

The main body 10 may be provided with the controller 20. The controller 20 may be provided in the form of a keyboard, a button, a dial, a foot switch, or a foot pedal. When the controller 20 is a keyboard, the controller 20 may be provided at an upper portion of the main body 10. When the controller 20 is a foot switch or a foot pedal, the controller 20 may be provided at a lower portion of the main body 10. An inspector may control the operation of the ultrasonic imaging apparatus 1 through the controller 20.

The controller 20 may include a control panel 21 having a keyboard, buttons, dials, and the like. The control panel 21 may be mounted on the main body 10. The second display 32 may be disposed on the control panel 21. A first handle 22 may be provided on one side of the control panel 21. A user may move the ultrasonic imaging apparatus 1 by holding the first handle 22 and applying a force to the ultrasonic imaging apparatus 1.

The probe device 100 may be connected to the main body 10 by a probe connecting member 50. The probe connecting member 50 may include a cable 51 and a connector 52. One side of the cable 51 may be connected to the probe device 100 and the other side of the cable 51 may be connected to the connector 52. The connector 52 may be detachably mounted to a connection portion 11 provided in the main body 10. Accordingly, the probe device 100 may be connected to the main body 10.

The ultrasonic imaging apparatus 1 may include a holder 23 formed on the control panel 21 so that a portable ultrasonic probe (not shown) may be mounted on the main body 10.

The main body 10 may include a second handle 12. The user may hold the second handle 12 and move the ultrasonic imaging apparatus 1. The second handle 12 may be disposed in the rear of the main body 10.

The main body 10 may include a plurality of castors 13 disposed at a lower portion of the main body 10 so as to move the ultrasonic imaging apparatus 1. The castors 13 may be provided to move the main body 10 in a specific direction, to be freely movable, or to be locked so that the main body 10 stops at a specific position.

The castors 13 may be controlled by a castor manipulator 14 provided in the main body 10. The castor manipulator 14 may be provided in the form of a foot pedal as shown in FIG. 1, or may be provided in the form of a button, a dial, or the like. The user may depress and operate the foot pedal with a foot and hold the first handle 22 and/or the second handle 12 to move or stop the ultrasonic imaging apparatus 1.

The probe device 100 may include a probe 120 connected to the main body 10 and capable of irradiating and receiving ultrasonic waves to and from the inspection object, and a probe driving device 110 provided to move the probe 120.

The correction member 130 may correct the inspection object including a curved surface such as a breast to facilitate inspection.

A fixing member 140 may apply a force to the correction member 130 mounted on the inspection object so that the correction member 130 is brought into close contact with the inspection object.

Details of the probe device 100, the correction member 130, and the fixing member 140 will be described later.

The ultrasonic imaging apparatus 1 may include an inspection object seating member 150 on which an inspection object is placed.

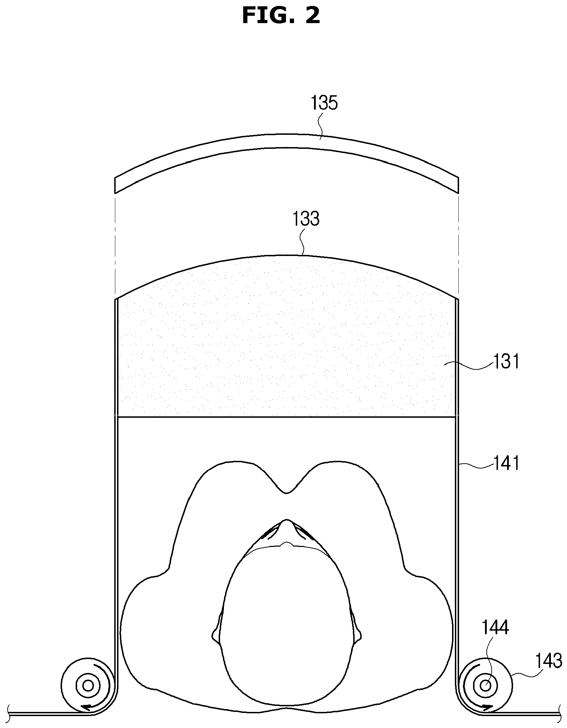

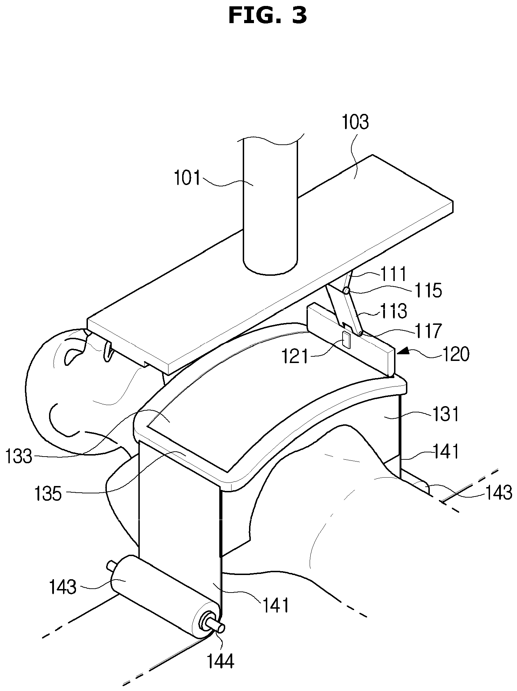

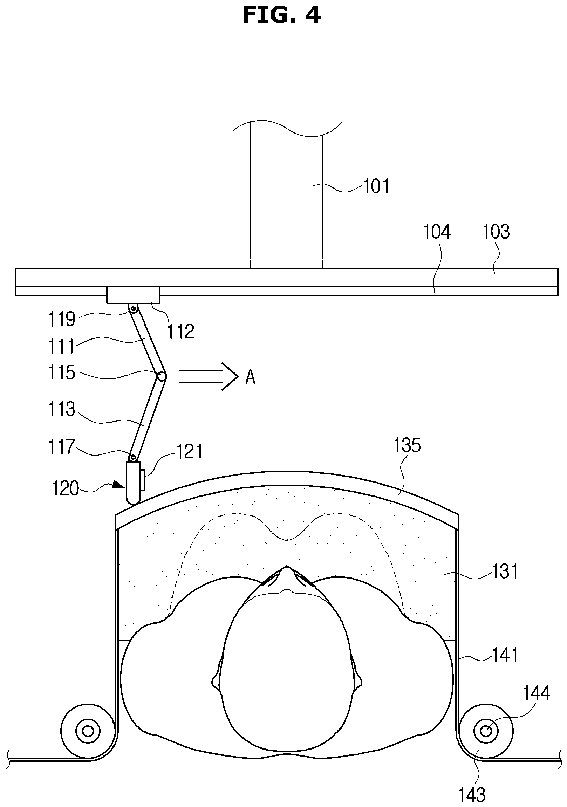

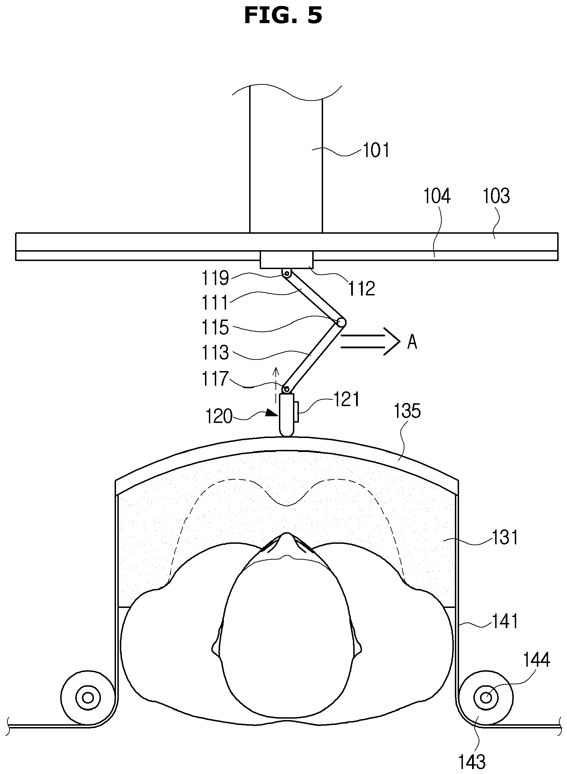

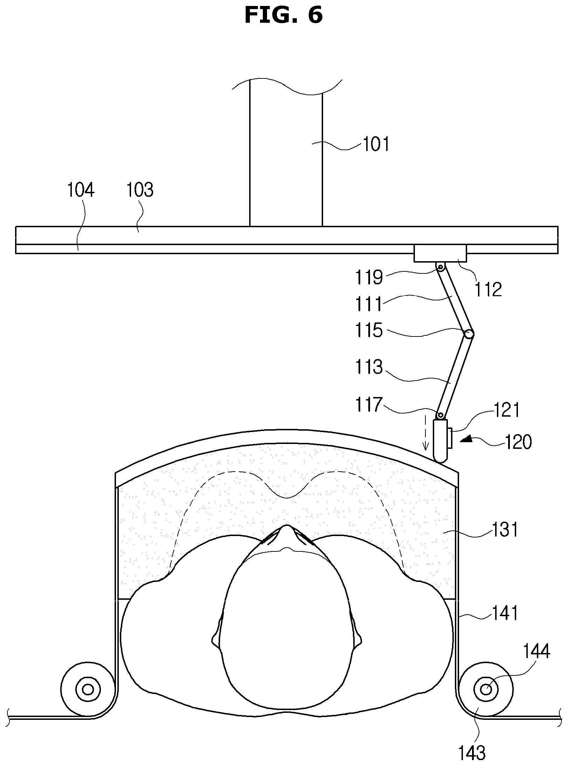

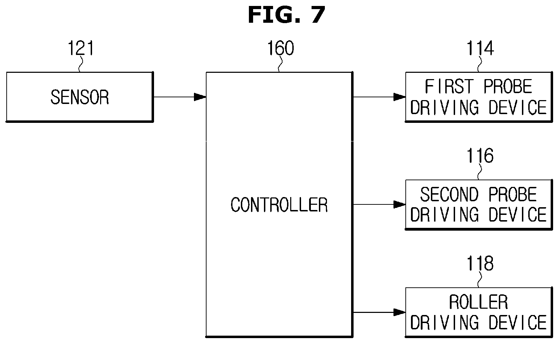

FIG. 2 is a view illustrating a state in which a correction member shown in FIG. 1 is mounted on an inspection object. FIG. 3 is a view illustrating a probe device, the correction member and a fixing member shown in FIG. 1. FIGS. 4 to 6 are views sequentially illustrating the operation of the probe device shown in FIG. 3. FIG. 7 is a control block diagram of the probe device shown in FIG. 3.

The probe device 100, the correction member 130, and the fixing member 140 will be described below with reference to FIGS. 2 to 7.

The probe device 100 may include a fixed body 101 connected to the cable 51. The fixed body 101 may be disposed at an upper portion of the inspection object seating member 150. The fixed body 101 may be movably provided, or may be movable within a predetermined range.

A guide body 103 may be disposed at a lower end of the fixed body 101. The guide body 103 may guide the probe driving device 110 so that the probe driving device 110 may scan the inspection object. To this end, the probe driving device 110 may include a driving source (not shown).

The probe driving device 110 may be configured to slidingly move the probe 120 on the correction member 130. The probe driving device 110 may move the probe so that the probe 120 is brought into close contact with the correction member 130 corresponding to the information measured by a sensor 121.

The probe driving device 110 may include a movement guide 104, a first driving member 111 slidably coupled to the movement guide 104, and a second driving member 113 rotatably coupled to the first driving member 111.

The movement guide 104 may be formed on the bottom surface of the guide body 103. The movement guide 104 may form a path through which the first driving member 111 moves. The movement guide 104 may extend in the left and right direction along the movement path of the probe 120.

The first driving member 111 may be slidably coupled to the movement guide 104 in a state of being coupled to the driving guide 112. However, the driving guide 112 may be omitted, and the first driving member 111 may be slidably coupled to the movement guide 104 directly. The first driving member 111 may be rotatably coupled to the driving guide 112 about a first rotating shaft 119. One end of the first driving member 111 is rotatably coupled to the driving guide 112 and the other end opposite to the one end may be rotatably coupled to the second driving member 113.

The second driving member 113 may be rotatably coupled to the first driving member 111. The second driving member 113 may be rotatably coupled to the first driving member 111 about a second rotating shaft 115. One end of the second driving member 113 may be rotatably coupled to the first driving member 111 and the other end opposite to the one end may be rotatably coupled to the probe 120.

The probe 120 may be coupled to the second driving member 113. The probe 120 may be rotatably coupled to the second driving member 113 about a third rotational shaft 117. As the probe driving device 110 drives, the probe 120 may be moved to scan the inspection object. The probe 120 may be moved in the left and right direction (direction A) by the probe driving device 110. The probe 120 may also be moved in the vertical direction by the probe driving device 110. The probe 120 may be moved while being kept in contact with a probe contact portion 133 by the probe driving device 110.

The probe driving device 110 may include a first probe driving device 114 for moving the first driving member 111 on the movement guide 104. The first probe driving device 114 may include a driving source (not shown) and a power transmitting member (not shown). The probe 120 may be moved in the left and right direction by the first probe driving device 114.

The probe driving device 110 may include a second probe driving device 116 for rotating the second driving member 113 with respect to the first driving member 111. The second probe driving device 116 may include a driving source (not shown) and a power transmitting member (not shown). The probe 120 may be moved in the vertical direction by the second probe driving device 116.

The probe 120 may be configured to be able to irradiate and receive ultrasonic waves to and from the inspection object. The probe 120 may be connected to the probe driving device 110 and may move in the left and right direction and/or the vertical direction. Since the probe 120 is well known, a detailed description thereof will be omitted.

The probe 120 may be provided with the sensor 121. The sensor 121 may measure a distance between the probe 120 and the correction member 130 and/or measure a force applied to the correction member 130 by the probe 120. The sensor 121 may be a force sensor or a pressure sensor. The sensor 121 may also be an infrared sensor or an optical sensor.

The position where the sensor 121 is disposed is not limited to the probe 120. For example, the sensor 121 may be disposed at any position, as long as it is capable of measuring the distance between the probe 120 and the correction member 130 and/or measuring the force applied to the correction member 130 by the probe 120.

The sensor 121 may transmit the measured information to the controller 160. The controller 160 may drive the first probe driving device 114 and/or the second probe driving device 116 based on the information received from the sensor 121.

Specifically, when a distance between the probe 120 and the probe contact portion 133 measured by the sensor 121 is longer than a preset distance or when a force applied to the probe contact portion 133 by the probe 120 is smaller than a preset force, the controller 160 may drive the second probe driving device 116 so that the probe 120 approaches the probe contact portion 133. That is, the second probe driving device 116 may rotate the second driving member 113 in a direction in which the probe 120 approaches the probe contact portion 133.

On the other hand, when the distance between the probe 120 and the probe contact portion 133 measured by the sensor 121 is shorter than the preset distance or when the force applied to the probe contact portion 133 by the probe 120 is larger than the preset force, the controller 160 may drive the second probe driving device 116 so that the probe 120 is away from the probe contact portion 133. That is, the second probe driving device 116 may rotate the second driving member 113 in a direction in which the probe 120 is away from the probe contact portion 133.

The correction member 130 may correct an inspection object having a curved surface such as a breast to be in a condition easy to inspect. The correction member 130 may be formed by forming an outer shape with a material that may pass ultrasonic waves such as latex, natural rubber or silicone and filling a liquid such as water that may pass ultrasonic waves or a gel for ultrasonic waves into the outer shape so that the probe 120 may inspect the inspection object. However, the material to be filled in the inside of the correction member 130 is not limited to a liquid, and any material may be used as long as it may pass ultrasonic waves and change the shape depending on the inspection object. The correction member 130 may include an inspection object contact portion 131 and the probe contact portion 133.

The inspection object contact portion 131 may be provided to be brought into close contact with the inspection object and deformable according to the shape of the inspection object. Accordingly, when the correction member 130 is mounted on the inspection object having a curved surface such as a breast, the pressing force applied to the inspection object may be minimized, and the inspection object contact portion 131 may be deformed while maintaining the shape of the inspection object.

The probe contact portion 133 is a portion that is in close contact with the probe 120, and may have a curved surface. Thus, the distance between the probe 120 and the inspection object having a curved surface such as a breast may be minimized. The probe 120 may be slidably moved on the curved surface of the probe contact portion 133. A porous membrane sheet may be disposed on the probe contact portion 133 so that a liquid gel for holding the probe 120 in close contact may be maintained.

A probe guide 135 may be disposed at an upper portion of the probe contact portion 133. The probe guide 135 may be made of a material harder than the probe contact portion 133. The probe guide 135 may guide the probe 120 so that the probe 120 moves along a hollow portion formed therein. By the probe guide 135, the probe 120 may move to scan a preset area.

According to the above configuration, the ultrasonic imaging apparatus 1 may more easily and accurately inspect the inspection object having a curved surface because the ultrasonic imaging apparatus 1 may correct the inspection object having a curved surface such as a breast to a curved surface (that is, the probe contacting portion 133) that is easy to inspect using the correction member 130.

The ultrasonic imaging apparatus 1 may include the fixing member 140 for applying a force to the correction member 130 so that the correction member 130 and the inspection object come in close contact with each other. The fixing member 140 may include a belt 141 connected to the correction member 130 and a roller 143 configured to apply tension to the belt 141.

The belt 141 may be connected to opposite sides of the correction member 130. The belt 141 may be fixed to the probe guide 135 of the correction member 130. The belt 141 may be disposed such that a portion of the belt 141 contacts the roller 143. The belt 141 may be made of a material having elasticity.

The roller 143 may contact a portion of the belt 141. The roller 143 may be provided to be rotatable about a roller rotation shaft 144. The fixing member 140 may include a roller driving device 118 for rotating the roller 143. The roller driving device 118 may rotate the roller 143 by the controller 160. Specifically, when the correction member 130 is mounted on the inspection object, the controller 160 may drive the roller driving device 118 configured to rotate the roller 143 so as to apply tension to the belt 141. The roller 143 may include a handle (not shown) to manually rotate the roller 143.

The roller 143 may be fixed so as not to be rotated by a brake (not shown). That is, when the belt 141 applies tension to the correction member 130 and the correction member 130 is brought into close contact with the inspection object, the roller 143 may be fixed so as not to be rotated by the brake.

When the correction member 130 is separated from the inspection object after the inspection is finished, the roller 143 may be rotated in a direction of releasing the tension of the belt 141. As a result, the belt 141 becomes loose, and the correction member 130 may be separated from the inspection object.

Although the belt 141 and the roller 143 are provided on opposite sides of the correction member 130 in this embodiment, the present disclosure is not limited thereto. For example, the belt 141 and the roller 143 may be provided only on one side of the correction member 130, and the other side may be simply fixed to the inspection object seating member 150.

According to the above-described configuration, the ultrasonic imaging apparatus 1 according to the embodiment of the present disclosure may shorten the inspection time because the ultrasonic imaging apparatus 1 may entirely scan the inspection object that has been corrected in a state that may be easily inspected by the correction member 130 when the first probe driving device 114 of the probe driving device 110 drives the probe 120 once in the direction A.

In addition, the ultrasonic imaging apparatus 1 may obtain an image of a constant quality because the state in which the probe 120 is in close contact with the probe contact portion 133 may be maintained based on the distance between the probe 120 and the probe contact portion 133 measured by the sensor 121 and/or the magnitude of the force applied to the probe contact portion 133 by the probe 120.

In particular, the ultrasonic imaging apparatus 1 may prevent the image quality from becoming different according to the proficiency level of the inspector because the ultrasonic imaging apparatus 1 may automatically move the probe 120 through the probe driving device 110.

Further, the ultrasonic imaging apparatus 1 may reduce the pressure applied to the inspection object because the correction member 130 is provided to be deformable according to the shape of the inspection object.

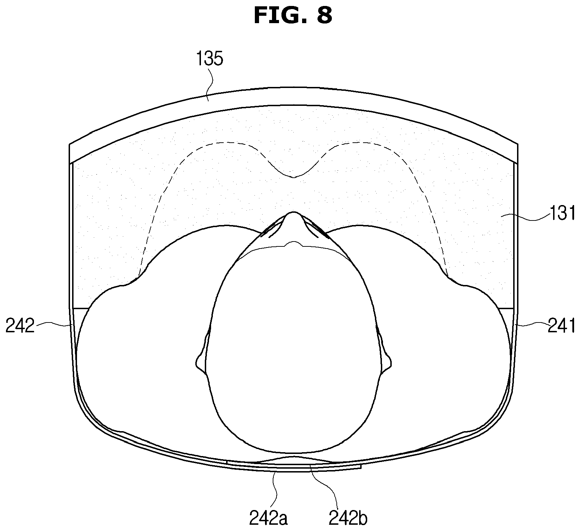

FIG. 8 is a view illustrating fixing members according to another embodiment of the present disclosure.

Fixing members 241 and 242 according to another embodiment of the present disclosure will be described below with reference to FIG. 8. In describing the embodiment shown in FIG. 8, the same components as those shown in FIGS. 1 to 7 are denoted by the same reference numerals and description thereof may be omitted.

Referring to FIG. 8, the fixing members 241 and 242 for applying tension to the correction member 130 may include a first tension member 241 having a first connecting portion 242b and a second tension member 242 having a second connecting portion 242a.

The first tension member 241 and/or the second tension member 242 may be made of a material having elasticity. The first tension member 241 may be connected to one side of the correction member 130 and the second tension member 242 may be connected to the other end opposite to the one end of the correction member 130.

The inspector may make the correction member 130 closely contact the inspection object by connecting the first connecting portion 242b of the first tension member 241 and the second connecting portion 242a of the second tension member 242 after contacting the inspection object contact portion 131 to the inspection object. The first connecting portion 242b and the second connecting portion 242a may be provided with a connecting device such Velcro or a hook.

Alternatively, although not shown, the first tension member 241 and the second tension member 242 are integrally formed.

When the fixing members 241 and 242 are configured as shown in FIG. 8, the fixing members 241 and 242 may have a relatively simple structure as compared with the fixing member 140 shown in FIGS. 1 to 7.

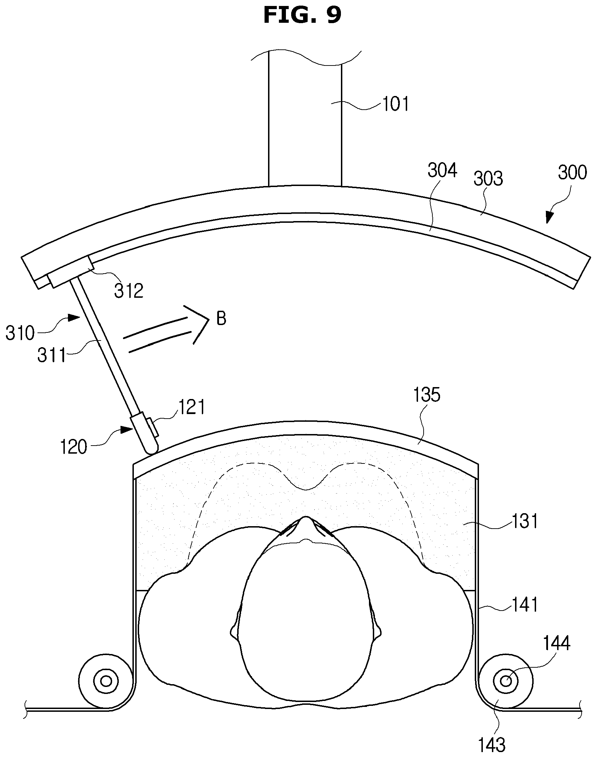

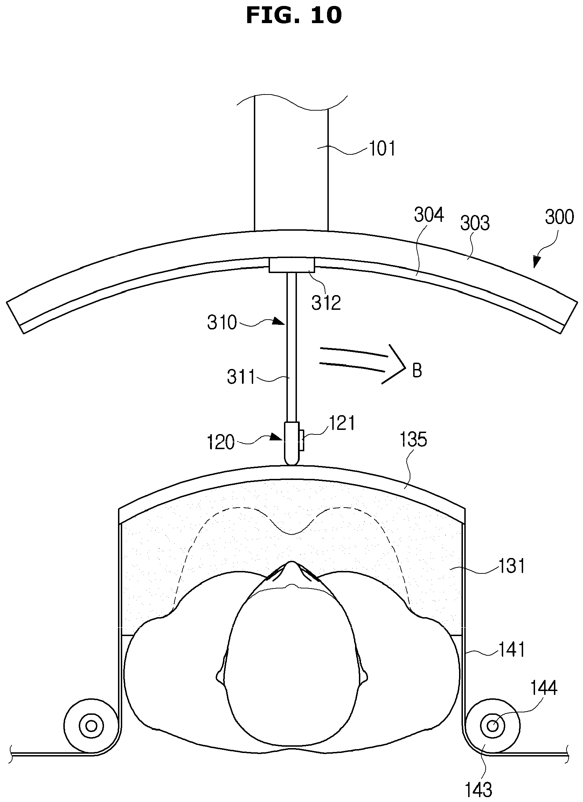

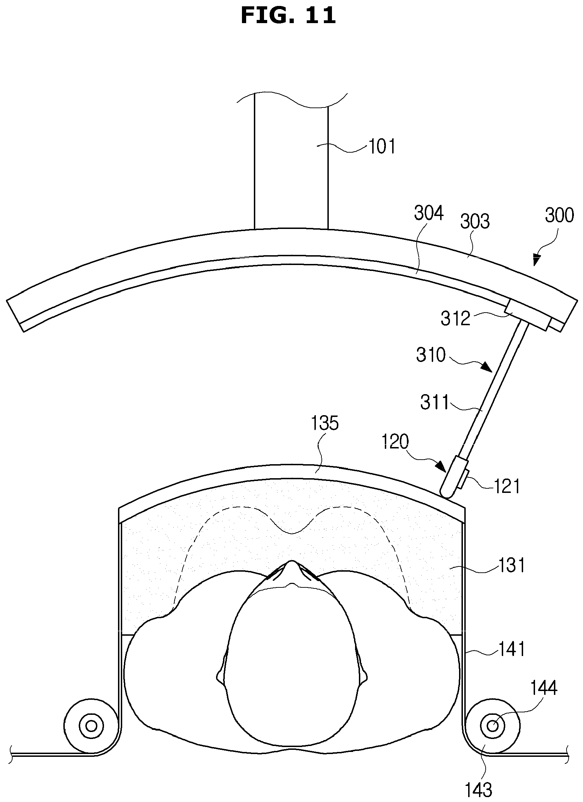

FIGS. 9 to 11 are views sequentially illustrating the operation of a probe device according to another embodiment of the present disclosure.

A probe device 300 according to another embodiment of the present disclosure will be described below with reference to FIGS. 9 to 11. In describing the embodiment shown in FIGS. 9 to 11, the same components as those shown in FIGS. 1 to 7 are denoted by the same reference numerals and description thereof may be omitted.

Referring to FIGS. 9 to 11, a probe driving device 310 may include a movement guide 304 formed to have a curvature corresponding to a curvature of the probe contact portion 133, and a driving member 311 slidably coupled to the movement guide 304.

The movement guide 304 may be formed on the bottom surface of a guide body 303. The movement guide 304 may form a movement path of the driving member 311. Since the movement guide 304 is formed to have a curvature corresponding to the curvature of the probe contact portion 133, the distance between the movement guide 304 and the probe contact portion 133 may be the same everywhere along the movement path of the probe 120.

The driving member 311 may be coupled to the movement guide 304 in a state of being coupled to a driving guide 312. The driving member 311 may be formed to have a length such that the probe 120 may be brought into close contact with the correction member 130. As the driving guide 312 slidingly moves on the movement guide 304, the driving member 311 may move in the left and right direction (direction B). As the driving member 311 moves in the left and right direction, the probe 120 may move in the left and right direction and scan the inspection object.

The probe driving device 310 shown in FIGS. 9 to 11 is different from the probe driving device 110 shown in FIGS. 1 to 7 in that the second probe driving device for moving the probe 120 in the vertical direction may be omitted.

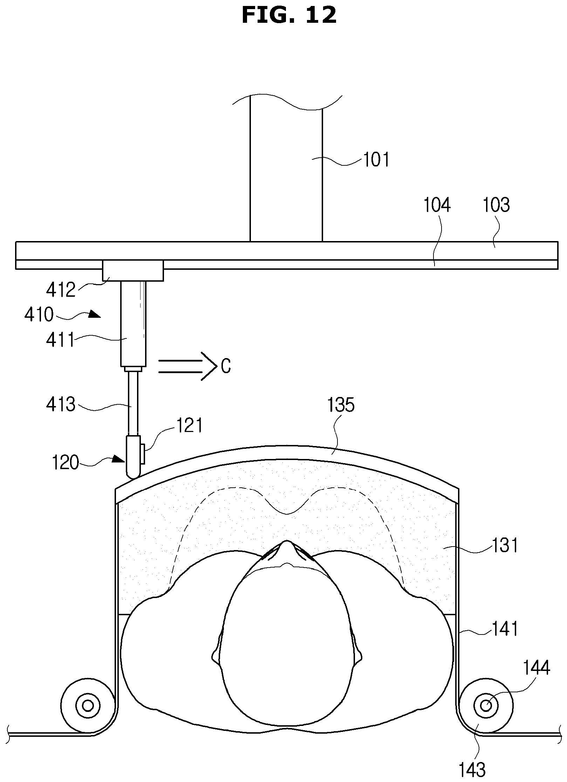

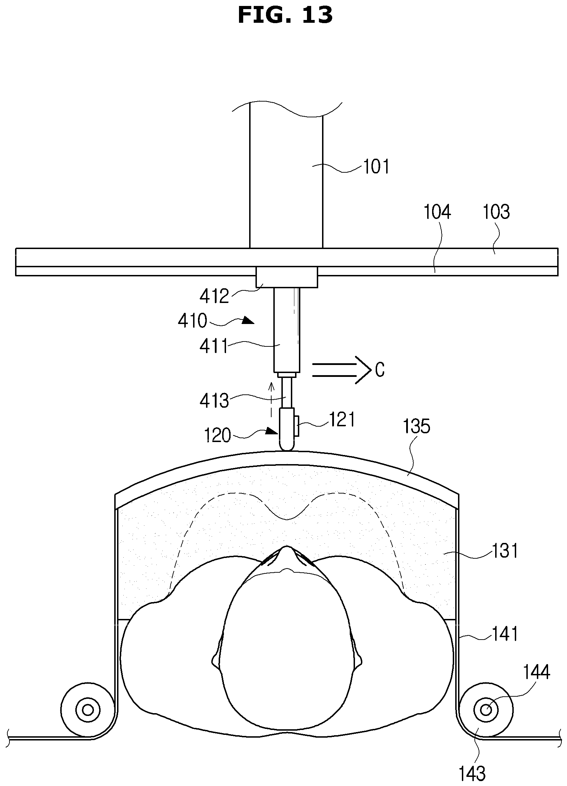

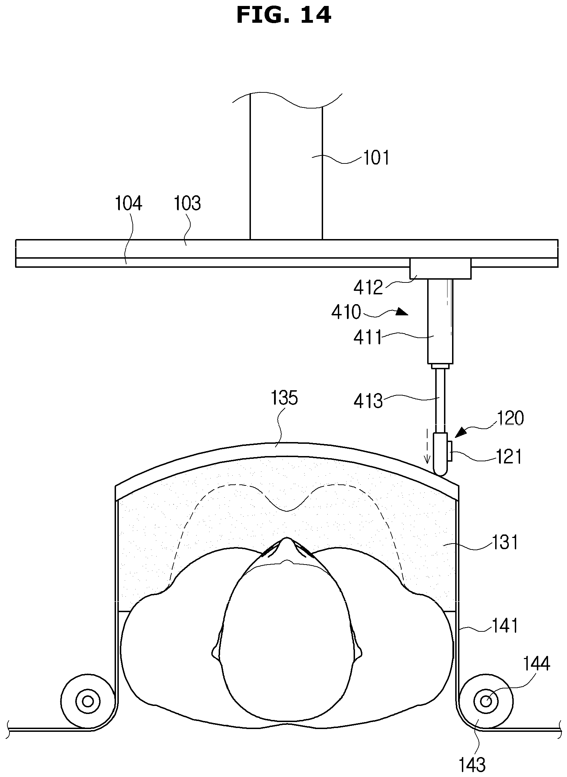

FIGS. 12 to 14 are views sequentially illustrating the operation of a probe device according to another embodiment of the present disclosure.

A probe driving device 410 according to another embodiment of the present disclosure will be described below with reference to FIGS. 12 to 14. In describing the embodiment shown in FIGS. 12 to 14, the same components as those shown in FIGS. 1 to 7 are denoted by the same reference numerals and description thereof may be omitted.

Referring to FIGS. 12 to 14, the probe driving device 410 may include the movement guide 104, a first driving member 411 slidably coupled to the movement guide 104, and a second driving member 413 slidably coupled to the first driving member 411.

The first driving member 411 may be slidably coupled to the movement guide 104 through a driving guide 412. The first driving member 411 may be moved in the left and right direction (direction C) by the first probe driving device 114.

The second driving member 413 may be slidably coupled to the first driving member 411. The second driving member 413 may be slidingly driven with respect to the first driving member 411 by the second probe driving device 116.

Specifically, when the distance between the probe 120 and the probe contact portion 133 measured by the sensor 121 is longer than the preset distance or the force applied to the correction member 130 by the probe 120 is smaller than the preset force, the controller 160 may drive the second probe driving device 116 to slide the second driving member 413 so that the probe 120 approaches the probe contact portion 133.

Conversely, when the distance between the probe 120 and the probe contact portion 133 measured by the sensor 121 is shorter than the preset distance or when the force applied to the probe contact portion 133 by the probe 120 is larger than the preset force, the controller 160 may drive the second probe driving device 116 to slide the second driving member 413 so that the probe 120 is away from the probe contact portion 133.

FIGS. 12 to 14 illustrate that the second driving member 413 slides into the first driving member 411, but the present disclosure is not limited thereto. For example, the probe driving device 410 may be configured such that the first driving member 411 slides into the second driving member 413.

According to the above configuration, the ultrasonic imaging apparatus 1 may acquire an image of a constant quality only by one scan because the probe 120 may scan the inspection object in a state of being brought into close contact with the correction member 130 and the correction member 130 may correct the inspection surface of the inspection object having a curved surface such as a breast to facilitate the scanning.

In addition, the ultrasonic imaging apparatus 1 may acquire an image of a constant quality regardless of the proficiency level of the inspector because the controller 160 drives the probe driving device 410 based on the information measured by the sensor 121 so that the probe 120 is automatically moved and scans the inspection object.

As is apparent from the above, the ultrasonic imaging apparatus according to an embodiment of the present disclosure can scan a portion of an inspection object formed as a curved surface such as a breast by one scan, thereby shortening the inspection time.

The ultrasonic imaging apparatus according to an embodiment of the present disclosure can perform the inspection while minimizing the pressure on a curved surface such as a breast, thereby reducing the pain felt by the subject.

The ultrasonic imaging apparatus according to an embodiment of the present disclosure can reduce a degree of intervention of the inspector, thereby obtaining an image of a constant quality.

The embodiments disclosed with reference to the accompanying drawings have been described above. It will be understood by those skilled in the art that various changes in form and details may be made therein without departing from the spirit and scope of the disclosure as defined by the appended claims. The disclosed embodiments are illustrative and should not be construed as limiting.

## Claims

1. An ultrasonic imaging apparatus comprising:
a probe to irradiate and receive ultrasonic waves;
a correction member including an inspection object contact portion provided to be in contact with an inspection object and to be deformable according to the shape of the inspection object, and a probe contact portion provided such that the probe is slidably moved and having a curved surface;
a sensor to measure a distance between the probe and the probe contact portion or a force applied to the probe contact portion by the probe; and
a probe driving device to slidingly move the probe on the curved surface of the probe contact portion and to move the probe so that the probe is brought in close contact with the probe contact portion corresponding to information measured by the sensor.

2. The ultrasonic imaging apparatus according to claim 1, wherein
the probe driving device includes a movement guide, a first driving member slidably coupled to the movement guide, and a second driving member rotatably coupled to the first driving member.

3. The ultrasonic imaging apparatus according to claim 2, wherein
the probe driving device further includes a first probe driving device to move the first driving member on the movement guide, and a second probe driving device to rotate the second driving member with respect to the first driving member.

4. The ultrasonic imaging apparatus according to claim 3, wherein the second probe driving device is configured to,
rotate the second driving member so that the probe approaches the probe contact portion when a distance between the probe and the probe contact portion measured by the sensor is longer than a preset distance or when a force applied to the probe contact portion by the probe is smaller than a preset force, and
rotate the second driving member so that the probe is away from the probe contact portion when the distance between the probe and the probe contact portion measured by the sensor is shorter than the preset distance or when the force applied to the probe contact portion by the probe is larger than the preset force.

5. The ultrasonic imaging apparatus according to claim 1, wherein
the probe driving device includes a movement guide, a first driving member slidably coupled to the movement guide, and a second driving member slidably coupled to the first driving member.

6. The ultrasonic imaging apparatus according to claim 5, wherein
the probe driving device further includes a first probe driving device to move the first driving member on the movement guide, and a second probe driving device to slide the second driving member with respect to the first driving member.

7. The ultrasonic imaging apparatus according to claim 6, wherein the second probe driving device is configured to,
slide the second driving member so that the probe approaches the probe contact portion when a distance between the probe and the probe contact portion measured by the sensor is longer than a preset distance or a force applied to the probe contact portion by the probe is smaller than a preset force, and
slide the second driving member so that the probe is away from the probe contact portion when the distance between the probe and the probe contact portion measured by the sensor is shorter than the preset distance or when the force applied to the probe contact portion by the probe is larger than the preset force.

8. The ultrasonic imaging apparatus according to claim 1, wherein
the probe driving device includes a movement guide formed to have a curvature corresponding to a curvature of the probe contact portion, and a driving member slidably coupled to the movement guide.

9. The ultrasonic imaging apparatus according to claim 1, wherein
the sensor is disposed in the probe.

10. The ultrasonic imaging apparatus according to claim 1, further comprising:
a fixing member to apply a force to the correction member so that the correction member and the inspection object are brought into close contact with each other.

11. The ultrasonic imaging apparatus according to claim 10, wherein
the fixing member includes a belt connected to the correction member, and a roller provided to be connected to the belt and apply a force to the belt so that the correction member is brought into close contact with the inspection object or apply a force to the belt so that the correction member is apart from the inspection object.

12. The ultrasonic imaging apparatus according to claim 10, wherein
the fixing member includes a tension member connected to the correction member and made of a material having elasticity.

13. The ultrasonic imaging apparatus according to claim 12, wherein
the tension member includes a first tension member having a first connecting portion, and a second tension member having a second connecting portion connected to the first connecting portion of the first tension member.

14. The ultrasonic imaging apparatus according to claim 1, wherein
the inside of the correction member is filled with a liquid passing ultrasonic waves.

15. The ultrasonic imaging apparatus according to claim 1, wherein
the correction member includes a probe guide disposed at the probe contact portion to guide the movement of the probe and made of a material harder than the probe contact portion.
